# EUROPÄISCHE PATENTSCHRIFT

(11) **EP 0 858 807 B1**
(45) Veröffentlichungstag und Bekanntmachung des Hinweises auf die Patenterteilung: **26.04.2000**
(21) Anmeldenummer: 97121009.1
(22) Anmeldetag: 29.11.1997
(51) Int. Cl.: A61K 31/685

(54) **Verwendung von Dopaminrezeptor-Antagonisten in der palliativen Tumortherapie**
Use of dopamine receptor antagonists in palliative tumor therapy
Utilisation d'antagonistes de récepteur de dopamine dans la thérapie anti-tumeurs palliative

(30) Priorität: 06.12.1996 DE 19650778
(43) Veröffentlichungstag der Anmeldung: 19.08.1998
(73) Patentinhaber: ASTA Medica Aktiengesellschaft, 01277 Dresden (DE)
(72) Erfinder: Nickel, Bernd, Dr., 64367 Mühltal (DE); Klenner, Thomas, Dr., 55218 Ingelheim (DE); Hilgard, Peter, Dr., 60325 Frankfurt (DE); Engel, Jürgen, Prof., 63755 Alzenau (DE)

(56) Entgegenhaltungen:
- EP-A- 0 855 397
- US-A- 4 707 484
- VERWEIJ, J. ET AL.: "A Dose-Finding Study of Miltefosine (Hexadecylphosphocholine) in Patients with Metastatic Solid Tumours" J. CANCER RES. CLIN. ONCOL., Bd. 118, 1992, Seiten 606-608, XP002089922
- NICKEL, B. ET AL.: "D-21266: A New Anti-tumour Alkylphosphocholine Derivative with less Side Effects" PROC. ANNU. MEET. AM. ASSOC. CANCER RES., Bd. 38, 1997, Seite A4102 XP002086940 US
- VERWEIJ J. ET AL: "Phase II Study of Oral Miltefosine in Patients with Squamous Cell Head and Neck Cancer" EUR. J. CANCER, Bd. 29A, Nr. 5, 1993, Seiten 778-779, XP002089921

## Beschreibung

Die Erfindung betrifft die Verwendung von Dopamin-rezeptor-Antagonisten in der palliativen Tumortherapie und zwar insbesondere Mittel zur Antagonisierung der Gewichtsabnahme , wie sie häufig bei der Tumortherapie durch Alkylphosphocholine und verwandte Strukturen beobachtet wird.

Es ist bekannt und beschrieben, daß das Alkylphospholipid Miltefosin bei Patienten bei der Behandlung von Krebsleiden Nebenwirkungen hervorruft. Diese äußern sich bei den mit Miltefosin behandelten Patienten auch durch einen deutlichen Körpergewichtsverlust. (Eur.J.Cancer, Vol.29 A, No.2, pp.208-209,1993)
Weitere Nebenwirkungen der klassischen Chemotherapie z.B. mit Alkyantien sind: Schädigung der Gewebe mit hoher Proliferationsrate, Leuko- und Thrombopenie, Abfall der Erythrozyten Magen- Darm Störungen, Appetitlosigkeit, Oberbauch-beschwerden, Resorptionsstörungen und Diarrhoe sowie Haarausfall ferner Leberschädigungen und Hyperurikämie.

In einer Dosisfindungsstudie von J. Verweij et. al. ( J. Cancer Res. Clin. Oncol (1992) 118:606-608) wurde in der Kombination mit Miltefosin beobachtet, daß die meisten Antiemetika (einschließlich 5HT3 Antagonisten ) inaktiv waren in der Verhinderung von Erbrechen und Übelkeit. Der geringste emetische Effekt wurde erzielt, wenn Miltefosin unmittelbar nach dem Essen genommen wurde, wobei Domperidon 0,5 Stunden vor dem Essen in einer Dosierung von 20 mg gegeben wurde. In der später durchgeführten Phase II Studien des gleichen Verfassers (Eur. J. Cancer Vol 29 A, Npo. 5 S. 778(1993) wurde indessen festgestellt, daß ein Erbrechen nicht verhindert werden konnte, weder durch Standard Antiemetika noch durch 5HT3 Antagonisten.

Da der Gewichtsverlust unter der Tumorbehandlung mit Alkylphosphocholinen zu einer weiteren Schwächung des ohnehin belasteten Organismus führt, war es Aufgabe der Erfindung, eine Substanz zu charakterisieren, die in Kombination mit der Verbindung die bekannte Gewichtsabnahme als Nebenwirkung der Alkylphosphocholine antagonisiert.
Dabei muß sichergestellt werden, daß die Antitumorwirkung durch die Kombination mit dem Antidot nicht aufgehoben oder abgeschwächt wird und keine zusätzlichen Nebenwirkungen durch die Gabe der Kombination auftreten.

Die Aufgabe wurde nun dahingehend gelöst , daß Domperidon und / oder Pimozid zur Herstellung eines Medikamentes zur Behandlung des Gewichtsverlustes bei Tumor-Patienten, die mit Octadecyl-(1,1-dimethylpiperidinio-4-yl)-phosphat, Versuchsbezeichnung D 21266, behandelt wurden, verwendet wird.
Dabei können Domperidon und / oder Pimozid mit dem o.g. Alkylphosphocholin in getrennter oder fixer Kombination vorliegen.
Zweckmäßigerweise kann das Mittel in folgendem Verhältnis verabreicht werden :
10 bis 120 mg / Tag, bevorzugt 30 mg / Tag Domperidon oder
2 bis 16 mg / Tag, bevorzugt 4mg / Tag Pimozid und
bis zu 300 mg / Tag Octadecyl-(1,1-dimethyl-piperidinio-4-yl)-phosphat.
Es versteht sich, daß diese Mittel sowohl in einer fixen Kombination mit dem Alkylphosphocholin als auch jeweils in Einzelpackungen und zeitversetzt verabreicht werden können.

### Beispiel

### Experimentelle Befunde

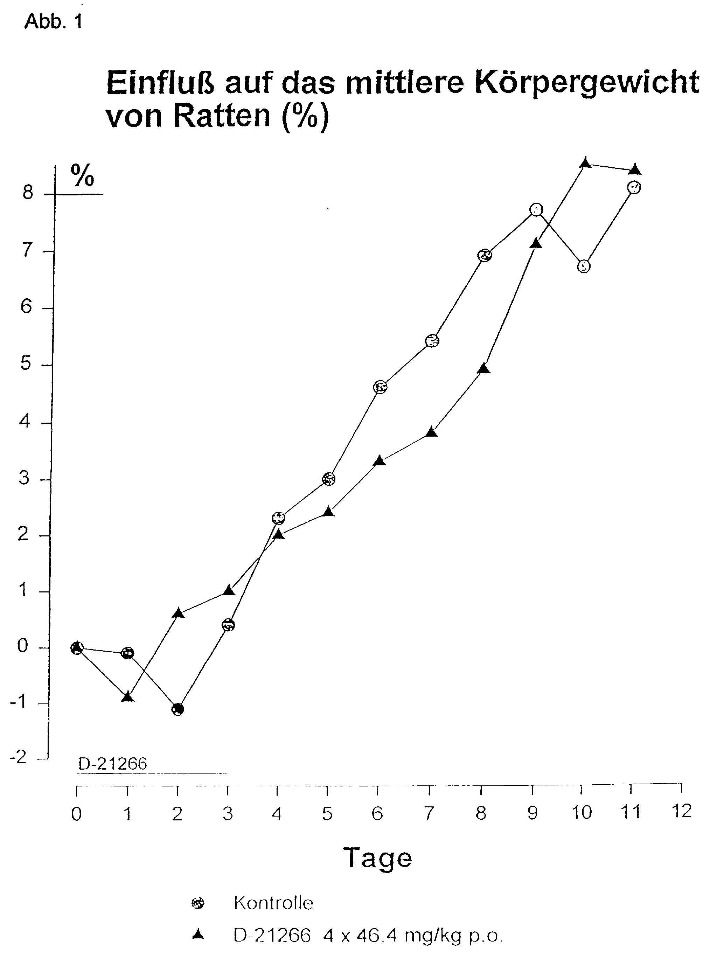

Es wurden vergleichende Untersuchungen nach alleiniger Gabe von Octadecyl-(1,1-dimethyl-piperidinio-4-yl)-phosphat ( D-21266) und in Kombination mit verschiedenen appetitanregenden Verbindungen bezüglich Körpergewichtsveränderungen an gesunden und tumortragenden Ratten untersucht.
Nach Gabe des Alkylphosphocholine-Derivates Octadecyl-(1,1-dimethyl-piperidinio-4-yl)-phosphat (D-21266) kommt es im Vergleich mit Miltefosin zu einem geringeren Gewichtsverlust bei den Tieren. Die Ratten nehmen weniger zu als die Kontrolltiere (Abb. 1).

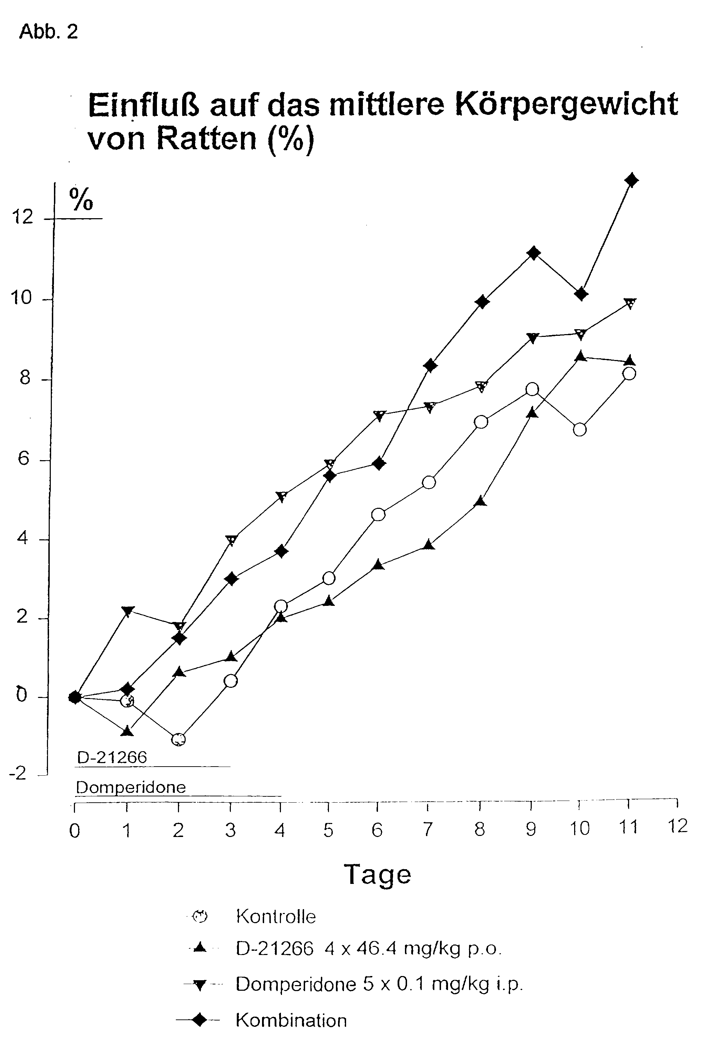

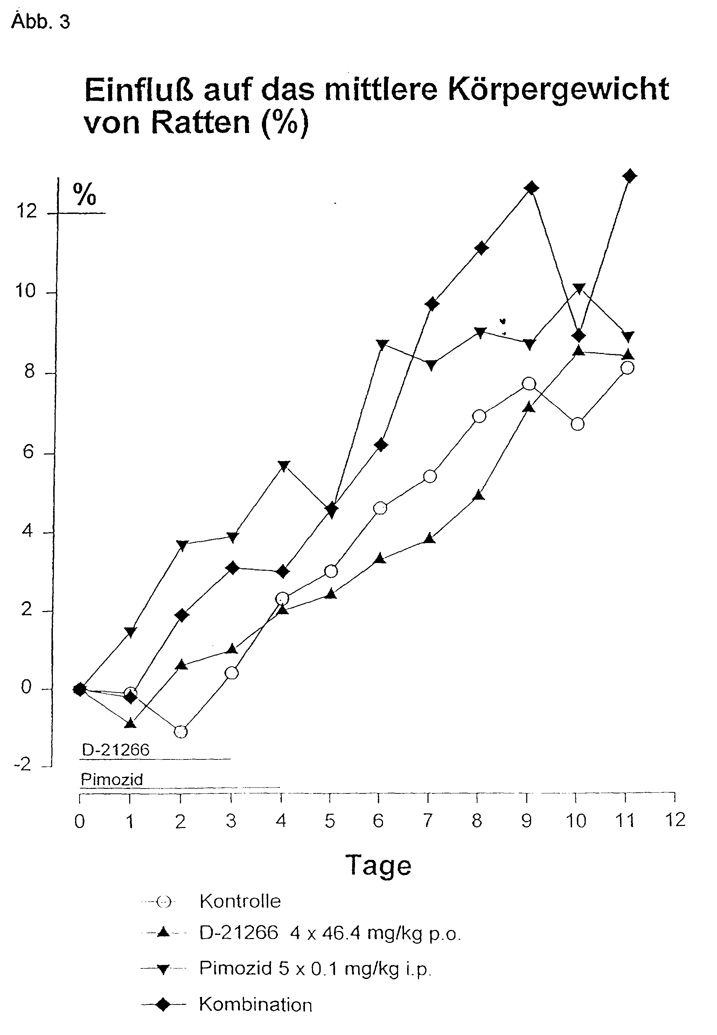

Durch die gleichzeitige Gabe der Dopamin-rezeptor-Antagonisten Domperidon oder Pimozid wird die langsamere Gewichtszunahme der mit Octadecyl-(1,1-dimethyl-piperidinio-4-yl)-phosphat behandelten Tiere mehr als kompensiert. Die Tiere sind sogar deutlich schwerer als die nur mit Placebo behandelten Kontrolltiere (Abb. 2 und 3).
Die beiden anderen appetitanregenden Verbindungen Cyproheptadin und Metoclopramid (Serotoninrezeptor-Antagonisten) hatten in der Kombination mit Octadecyl-(1,1-dimethyl-piperidinio-4-yl)-phosphat keinen signifikanten Einfluß auf die Körpergewichtsentwicklung der Tiere (Tab.1).

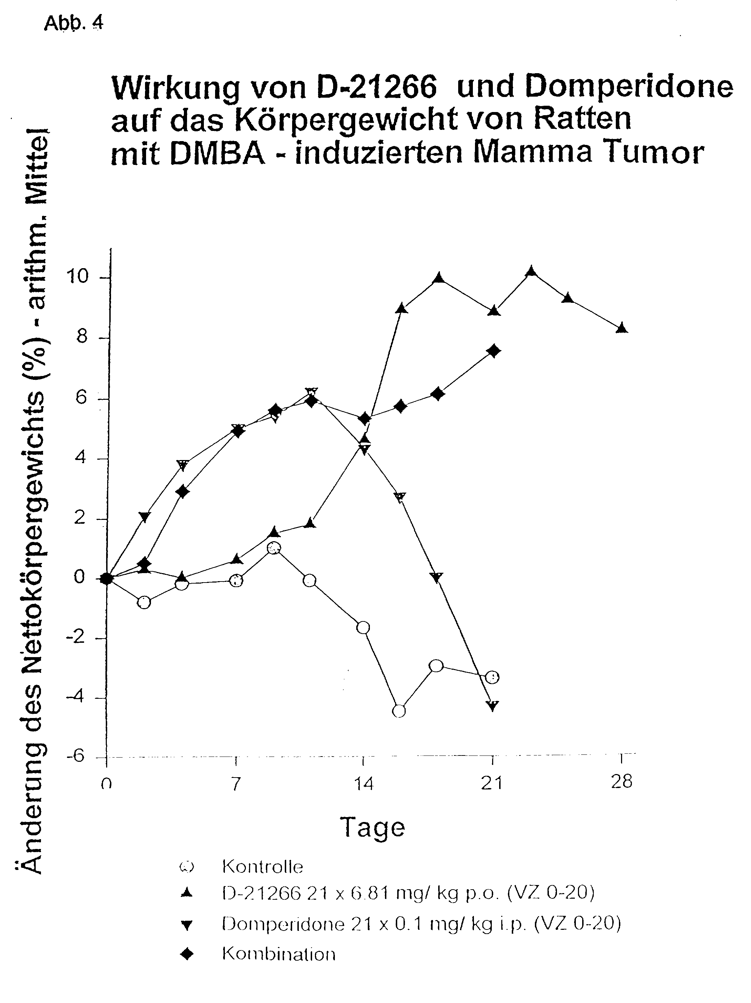

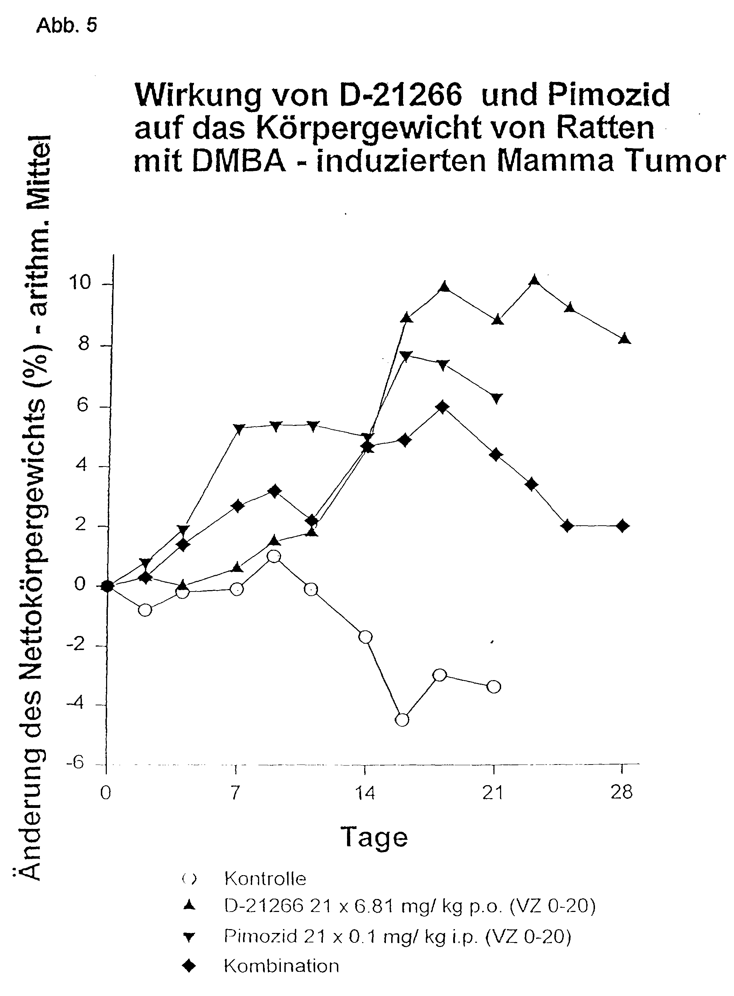

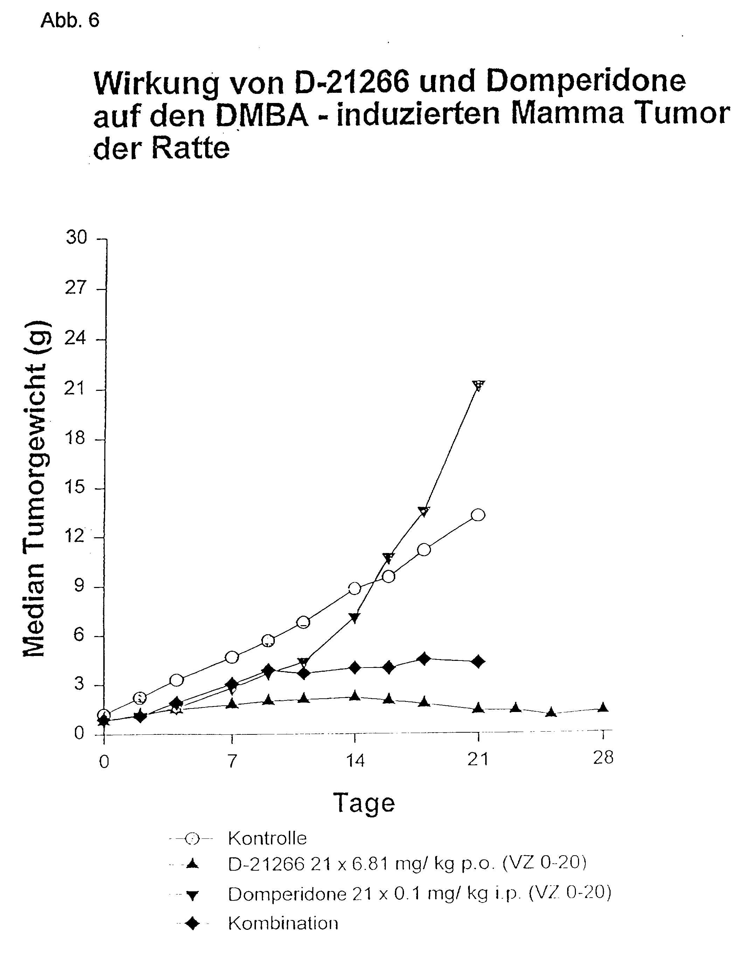

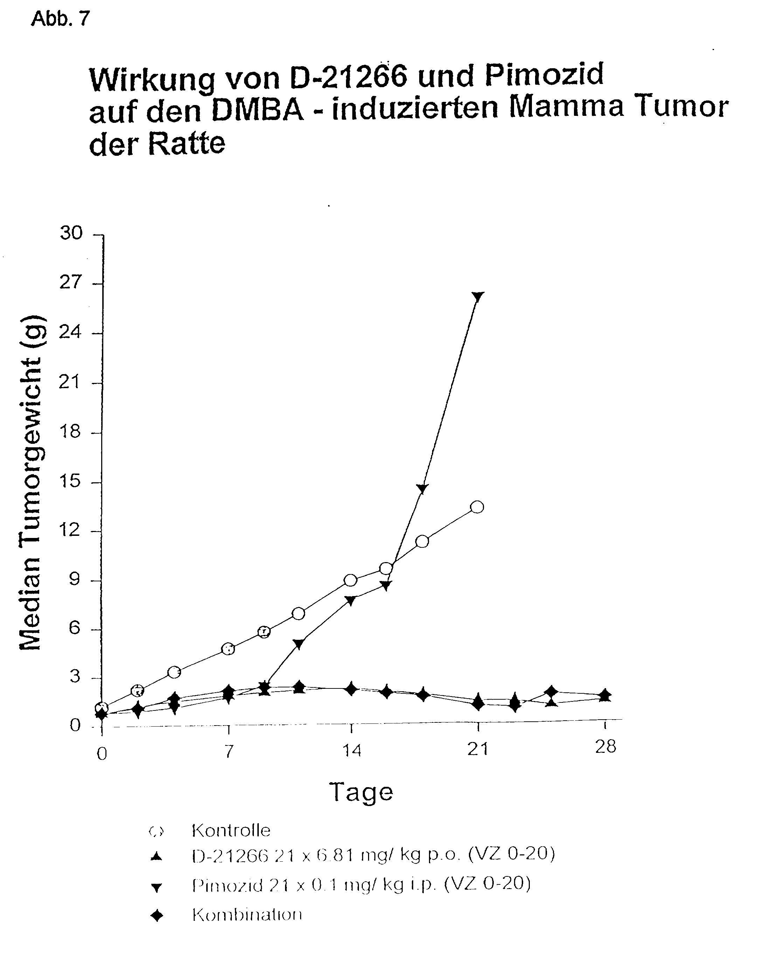

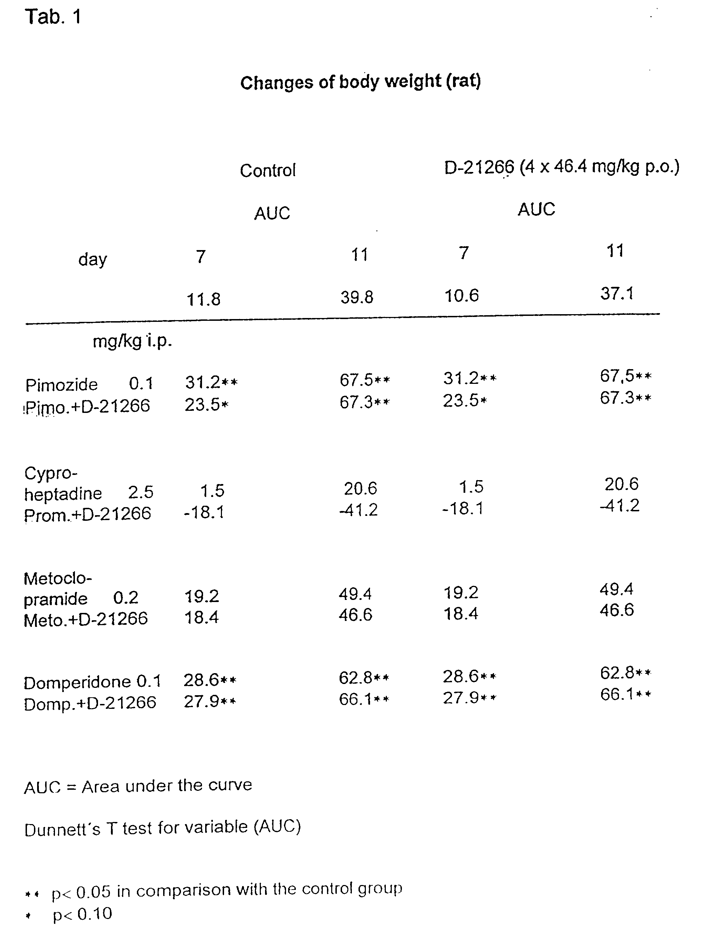

An tumortragenden Tieren ergibt sich das selbe Bild wie an gesunden Tieren. Domperidon und Pimozid alleine und in Kombination mit Octadecyl-(1,1-dimethyl-piperidinio-4-yl)-phosphat verstärken die Körpergewichtsentwicklung der Tiere ohne die Antitumorwirkung zu beeinflussen (Abb. 4-7).
Die Dosierungen für die Antidots wurden so gewählt, daß die Verbindungen selbst keinerlei Nebenwirkungen bei den Versuchstieren hervorrufen.

## Patentansprüche

1. Verwendung von Domperidon und / oder Pimozid zur Herstellung eines Medikamentes zur Antagonisierung von Nebenwirkungen bei der Tumortherapie mit Octadecyl-(1,1-dimethyl-piperidinio-4-yl)-phosphat, wobei Domperidon und / oder Pimozid mit Octadecyl-(1,1-dimethyl-piperidinio-4-yl)-phosphat entweder getrennt oder in fixer Kombination vorliegen können.

2. Verwendung nach Anspruch 1 dadurch gekenzeichnet, daß die Mittel in folgendem Verhältnis verabreicht werden :
10 bis 120 mg / Tag Domperidon oder
2 bis 16 mg / Tag, Pimozid und
bis zu 300 mg / Tag Octadecyl-(1,1-dimethyl-piperidinio-4-yl)-phosphat.

3. Verwendung nach Anspruch 2 dadurch gekenzeichnet, daß die Mittel in folgendem Verhältnis verabreicht werden :
30 mg / Tag Domperidon oder
4mg / Tag Pimozid und
bis zu 300 mg / Tag Octadecyl-(1,1-dimethyl-piperidinio-4-yl)-phosphat.

## Claims

1. Use of domperidone and/or pimozide for producing a medicament for antagonizing side effects in tumour therapy with octadecyl (1,1-dimethylpiperidinio-4-yl)phosphate, it being possible for domperidone and/or pimozide to be present with octadecyl (1,1-dimethylpiperidinio-4-yl)phosphate either separately or in a fixed combination.

2. Use according to Claim 1, characterized in that the agents are administered in the following proportion:
10 to 120 mg/day of domperidone or
2 to 16 mg/day of pimozide and
up to 300 mg/day of octadecyl (1,1-dimethyl-piperidinio-4-yl)phosphate.

3. Use according to Claim 2, characterized in that the agents are administered in the following proportion:
30 mg/day of domperidone or
4 mg/day of pimozide and
up to 300 mg/day of octadecyl (1,1-dimethyl-piperidinio-4-yl)phosphate.

## Revendications

1. Utilisation de la dompéridone et/ou du pimozide pour la préparation d'un médicament en vue d'antagoniser les effets secondaires au cours de la thérapie tumorale, avec l'octadécyl-(1,1-diméthylpipéridinio-4-yl)-phosphate, dans laquelle la dompéridone et/ou le pimozide, peuvent se présenter avec l'octadécyl-(1,1-diméthylpipédinio-4-yl)-phosphate soit séparés ou dans une combinaison fixe.

2. Utilisation selon la revendication 1,
caractérisée en ce que
les compositions sont administrées dans le rapport suivant :
- de 10 à 120 mg/jour de dompéridone ou
- de 2 à 16 mg/jour de pimozide et
- jusqu'à 300 mg/jour d'octadécyl-(1,1-diméthylpipéridinio-4-yl)-phosphate.

3. Utilisation selon la revendication 2,
caractérisée en ce que
les compositions sont administrées dans le rapport suivant :
- 30 mg/jour de dompéridone ou
- 4 mg/jour de pimozide et
- jusqu'à 300 mg/jour d'octadécyl-(1,1-diméthylpipéridinio-4-yl)-phosphate.
